(19) Europäisches Patentamt
European Patent Office
Office européen des brevets

(11) Veröffentlichungsnummer: **0 033 539**
**B1**

(12) # EUROPÄISCHE PATENTSCHRIFT

(45) Veröffentlichungstag der Patentschrift: **19.01.83**

(51) Int. Cl.³: **C 07 D 231/46**, C 12 Q 1/28

(21) Anmeldenummer: **81100737.6**

(22) Anmeldetag: **02.02.81**

(54) **Verwendung von Aminoantipyrin-Verbindungen und neue Aminoantipyrinderivative.**

(30) Priorität: **07.10.80 DE 3039207**
**05.02.80 DE 3004129**
**13.01.81 DE 3100807**

(43) Veröffentlichungstag der Anmeldung:
**12.08.81 Patentblatt 81/32**

(45) Bekanntmachung des Hinweises auf die Patenterteilung:
**19.01.83 Patentblatt 83/3**

(84) Benannte Vertragsstaaten:
**AT BE CH DE FR GB IT LI LU NL SE**

(56) Entgegenhaltungen:
**AT-B-150 301**
**DE-C-92 009**

(73) Patentinhaber: **BOEHRINGER MANNHEIM GMBH, Sandhofer Strasse 112-132 Postfach 31 01 20, D-6800 Mannheim 31-Waldhof (DE)**

(72) Erfinder: **Batz, Hans-Georg, Dr., Traubinger Strasse 63, D-8132 Tutzing (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verwendung von Aminoantipyrin-Verbindungen und neue Aminoantipyrinderivate

Die Erfindung betrifft die Verwendung von Aminoantipyrin-Verbindungen der allgemeinen Formel

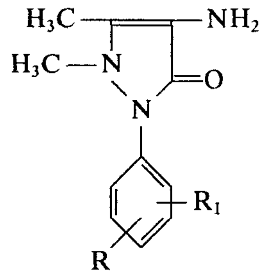

in der R die Gruppe $N(R_2)_2$, in der $R_2$ ein Wasserstoffatom, eine Alkylgruppe mit 1 bis 3 C-Atomen oder eine Acylgruppe mit 1 bis 3 C-Atomen bedeutet, darstellt und $R_1$ die gleiche Bedeutung wie R hat oder $R_1$ eine Alkylgruppe mit 1 bis 3 C-Atomen oder ein Wasserstoffatom ist, als Chromogene zur Messung der $H_2O_2$-Bildung bei enzymatischen Reaktionen in Gegenwart von Peroxidase und Puffer.

$H_2O_2$ ist das Reaktionsprodukt zahlreicher enzymatischer Reaktionen, an denen Oxidasen beteiligt sind. Solche Reaktionen, wie beispielsweise die Oxidation von Glycerin durch Glycerinoxidase oder von Cholesterin durch Cholesterinoxidase, sind für die Analytik und hier besonders für die medizinische Diagnostik von großer Bedeutung.

Bekannte Verfahren zur Bestimmung von enzymatisch gebildetem $H_2O_2$ basieren auf titrimetrischen, potentiometrischen, polarographischen und colorimetrischen Methoden, sowie auf enzymatischen Methoden, wobei die Enzyme Katalase oder Peroxidase verwendet werden. Bei der enzymatischen Bestimmung mittels Peroxidase werden als Indikatoren Chromogene eingesetzt, die mit $H_2O_2$ in Gegenwart der Peroxidase unter Bildung eines Farbstoffs reagieren, der photometrisch bestimmt werden kann. Ein bekanntes derartiges Reagenz zur $H_2O_2$-Bestimmung enthält das Indikatorsystem nach Trinder (Ann. Clin. Biochem. 6 [1969], 24–27), bei dem Phenol mit 4-Aminoantipyrin als Chromogene in Gegenwart von Peroxidase unter Einwirkung von $H_2O_2$ oxidativ zu einem Farbstoff gekuppelt werden, der photometrisch bestimmt wird. Anstelle von Phenol können auch andere phenolische Verbindungen verwendet werden.

Ein Nachteil des 4-Aminoantipyrins als Chromogen in der oben beschriebenen $H_2O_2$-Bestimmung besteht in einer mangelhaften Stabilität des gebildeten Farbtoffs. Da bei Testsystemen angestrebt wird, gegen den Reagenzienleerwert zu messen, bedeutet selbst eine scheinbar geringe Verbesserung der Farbstabilität einen großen Praktikabilitätsvorteil, z. B. durch die Ermöglichung längerer Ablesezeiten.

Der Erfindung liegt daher die Aufgabe zugrunde, Chromogene für die $H_2O_2$-Bestimmung anzugeben, die anstelle von 4-Aminoantipyrin verwendet werden können und bessere Farbstabilitäten der bei der oxidativen Kupplung mit einer phenolischen Verbindung gebildeten Farbstoffe aufzuweisen.

Gelöst wird diese Aufgabe durch die Verwendung der oben beschriebenen Verbindungen, bei denen es sich um Derivate des 4-Aminoantipyrins handelt. In diesen Verbindungen können die Substituenten R und $R_1$ jede Position im Phenylrest einnehmen. Bevorzugt werden aufgrund der leichteren Zugänglichkeit Verbindungen, in denen die Reste R und $R_1$ in p- oder/und o-Position stehen.

Die Herstellung der erfindungsgemäß verwendeten Verbindungen kann nach an sich bekannten Methoden erfolgen, beispielsweise durch Nitrierung von Antipyrin mit zwei Äquivalenten Salpetersäure unter Bildung von Dinitroantipyrin und Reduktion der Nitrogruppen zu den entsprechenden Aminogruppen, beispielsweise mittels Zinkstaub. Die Wasserstoffatome der phenylischen Aminogruppe lassen sich nach Maskierung der Aminogruppe in Position 4 des Antipyrins, beispielsweise durch Bildung der Schiff'schen Base mit Benzaldehyd, mit einem Alkylierungsmittel, wie Alkyljodid, oder mit einem Acylierungsmittel, z. B. Essigsäureanhydrid, alkylieren bzw. acylieren. Die Derivate, in denen R oder $R_1$ eine Alkylgruppe darstellt, werden zweckmäßig von den entsprechenden alkylierten Phenylpyrazolonen ausgehend in analoger Weise hergestellt.

Aufgrund der besseren Farbstabilität der Farbstoffe, die durch die erfindungsgemäß verwendeten Verbindungen bei der oxidativen Kupplung mit einer phenolischen Verbindung und $H_2O_2$ und Peroxidase gebildet werden, eignen sich die Verbindungen besonders zur Verwendung in Verfahren und Reagenzien zur enzymatischen Bestimmung von $H_2O_2$. Bei dieser Verwendung können die üblichen phenolischen Verbindungen, die mit 4-Aminoantipyrin unter Farbstoffbildung gekuppelt werden können, verwendet werden. Bevorzugt wird als phenolische Verbindung p-Chlorphenol. Beispiele für andere geeignete Verbindungen sind Phenol selbst, andere Phenolderivate, Anilinderivate, Naphthol, Naphtholderivate, Naphthylamin, Naphthylaminderivate, Aminochinoline, Hydroxychinoline, Dihydroxyphenylessigsäure und ähnliche. Die Umsetzung wird in gepufferter

Lösung durchgeführt. Als Puffersubstanzen und pH-Werte eignen sich die für Peroxidase bekannten diesbezüglichen Bedingungen. Bevorzugt werden pH-Werte zwischen 6 und 9. Im übrigen ist die Wahl des Puffers und des pH-Werts im Falle einer vorgeschalteten, $H_2O_2$ bildenden enzymatischen Reaktion vor allem durch die Anforderungen hinsichtlich Puffer und pH-Wert der daran beteiligten Enzyme bedingt. Diese Bedingungen sind sämtlich dem Fachmann bekannt und bedürfen daher hier keiner näheren Erläuterung.

Unter den erfindungsgemäß verwendeten Verbindungen der allgemeinen Formel sind diejenigen, bei denen R die Gruppe $N(R_2)_2$ bedeutet, in der jedes $R_2$ eine Alkylgruppe mit 1 bis 3 C-Atomen oder eine Acylgruppe mit 1 bis 3 C-Atomen bedeutet und ein $R_2$ auch ein Wasserstoffatom sein kann und $R_1$ die gleiche Bedeutung wie $R_2$ hat, wobei jedoch jedes $R_2$ ein H-Atom sein kann, oder eine Alkylgruppe mit 1 bis 3 C-Atomen oder ein Wasserstoffatom ist, neu und als neue Verbindungen ein weiterer Gegenstand der Erfindung.

Ein Reagenz zur Bestimmung von $H_2O_2$ auf Basis Peroxidase, wenigstens einer erfindungsgemäß verwendeten Verbindung, wenigstens einer phenolischen Verbindung und Puffersubstanz kann zusätzlich noch übliche Lösungsmittel, Stabilisatoren oder/und oberflächenaktive Substanzen enthalten. Besonders geeignet erwiesen sich dabei folgende Mengenverhältnisse der essentiellen Bestandteile dieses Reagenz:

0,5 bis 100 U/ml Peroxidase,
0,05 bis 20 mMol/l Verbindung der allgemeinen Formel,
0,5 bis 50 mMol/l phenolische Verbindung.

Oberflächenaktive Mittel werden, wenn sie zugegen sind, vorzugsweise in Mengen von 0,001 bis 0,1 g/ml, bezogen auf gebrauchsfertige Reagenzlösungen, eingesetzt.

Die folgenden Beispiele erläutern die Erfindung weiter.

Beispiel 1

A. Dinitroantipyrin

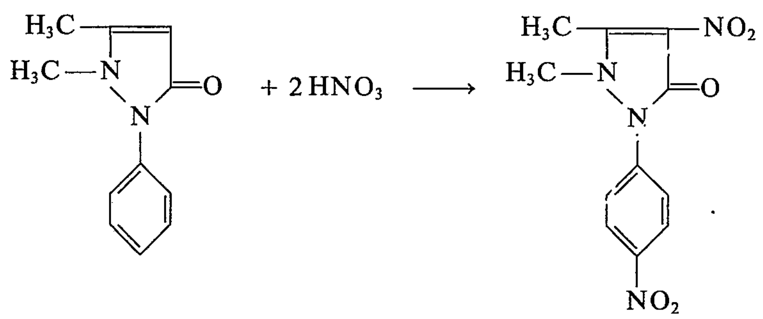


$C_{11}H_{12}N_2O$ $C_{11}H_{10}N_4O_5$

Chemikalien:

5 g (≙ 0,0268 mol) Antipyrin
3,7 ml = 3,38 g (≙ 2 × 0,0268 mol) 65% $HNO_3$
36 ml konzentrierte Schwefelsäure

Ausführung:

5 g Antipyrin in 30 ml konzentrierter $H_2SO_4$ lösen, dazu 3,7 ml konzentrierte $HNO_3$ und 6 ml $H_2SO_4$ (Antipyrin erhitzt sich beim Lösen in $H_2SO_4$ auf ~40° C),
$HNO_3/H_2SO_4$ unter Eiskühlung eintropfen (Temperatur max. +10° C). Anschließend langsam erwärmen und 30 Minuten auf kochendem Wasserbad halten (Ansatz wird immer dunkler), abkühlen, auf Eis gießen (~1 l), roter Niederschlag, absaugen, aus Eisessig umkristallisieren.

Ausbeute: 4,9 g

B. Diaminoantipyrin

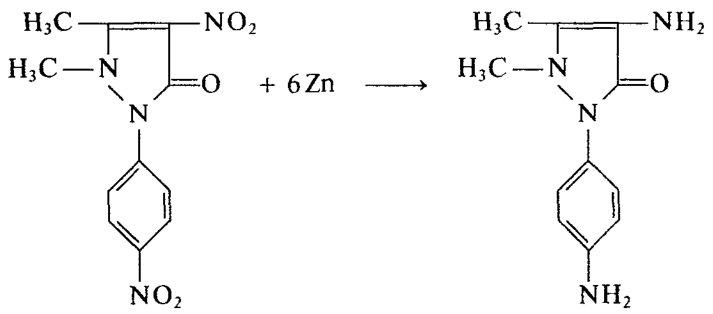

Chemikalien:

4 g Dinitroantipyrin ( ≙ 0,0144 mol)
8 g Zinkstaub ( ≙ 0,123 mol)

Ausführung:

4 g Dinitroantipyrin in 50 ml konzentrierter HCl lösen unter Kühlung (max. 20° C), Zugabe von Zinkstaub (~8 g) bis Lösung farblos ist. Mit 40%iger NaOH/NaOH-Plätzchen bis pH 7, dicker Niederschlag fällt aus, läßt sich schlecht absaugen (mit $CHCl_3$ läßt sich nicht mehr viel extrahieren).
Mutterlauge auf pH 11,5 stellen mit $CHCl_3$ extrahieren, einengen. Probe löst sich in $H_2O$, gibt mit DCP/$FECl_3$ dunkelrote Farbe.

Ausbeute: 0,4 g

Beispiel 2

A. Herstellung der 4-Benzalverbindung des
1-(p-Aminophenyl)-2,3-dimethyl-4-amino-pyrazolon-5 (II)

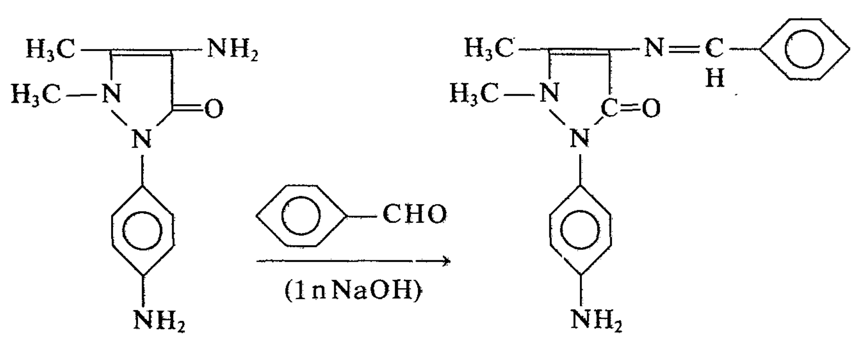


I

1,09 g (5 mmol) Diaminoantipyrin (I) werden in 15 ml $H_2O$ gelöst und mit 1 n Natronlauge auf pH 10 eingestellt. Dazu werden 0,53 ml (5 mmol) Benzaldehyd gegeben. Die Mischung wird 20 Stunden bei Raumtemperatur kräftig gerührt. Danach wird das kristalline Produkt abgesaugt und mit Wasser und Äther nachgewaschen.
Ausbeute: 82%.
Die erhaltene Substanz (II) wird ohne weitere Reinigungsschritte für die folgenden Umsetzungen verwendet.

B. 4-Benzalverbindung des 1-(p-Diäthylamino-phenyl)-2,3-dimethyl-4-amino-pyrazolon-5

9,18 g 4-Benzalverbindung des Diaminoantipyrins (0,03 mol) werden unter Rückfluß in
250 ml Äthylenglykoldimethyläther gelöst. Anschließend wird auf 60° abgekühlt. Danach erfolgt die

Zugabe von
56 g Kaliumcarbonat sowie
14,6 ml = 28,0 g Äthyljodid (0,18 mol).

Es wird 9 Stunden unter Rückfluß gekocht, und danach vom Kaliumcarbonat abgesaugt. Das Filtrat wird im Vakuum zur Trockne gebracht.

Rückstand: 12,16 g
Der Rückstand wird über eine Kieselgelsäule chromatographiert (Äthanol). Die chromatographisch reinsten Fraktionen werden aus Diäthyläther zur Kristallisation gebracht.

Ausbeute: 3,1 g
DC-Befund: einheitlich.

C. 1-(p-Diäthylamino-phenyl)-2,3-dimethyl-4-amino-pyrazolon-5

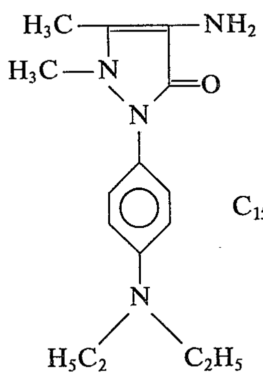

$C_{15}H_{22}N_4O$ MG 274

1,0 g des Produkts von Stufe B werden in 35 ml Salzsäure (2 n) bei Raumtemperatur gelöst und anschließend 2,5 Stunden verrührt. Die eintretende Benzalverbindung-Spaltung wird chromatographisch verfolgt. Zur Abtrennung des Benzaldehyds wird dreimal mit je 30 ml Chloroform ausgerührt. Danach wird die saure, wäßrige Phase mit Natronlauge (33%) auf den pH-Wert 10,0 gebracht und erneut mit Chloroform (dreimal je 30 ml) extrahiert. Das Chloroform wird im Vakuum abgezogen.

Rückstand: 0,855 g
Dieser wird einer erneuten Kieselgelbehandlung unterzogen (Chloroform/Äthanol 1 : 1).
Die Ausbeute an chromatographisch einheitlicher Substanz beträgt 0,356 g.
MS: 274

Beispiel 3

A. 4-Benzalverbindung des 1-(p-Acetamino-phenyl)-2,3-dimethyl-4-aminopyrazolon-5

15,3 g Benzalverbindung des Diaminoantipyrins (1/20 Mol) werden in 60 ml Dimethylformamid bei 45 bis 50° gelöst. Bei Raumtemperatur erfolgt die Zugabe von 10,2 g Acetanhydrid. Dabei steigt die Temperatur auf 50° an, und es tritt spontane Kristallisation ein. Nach 2 Stunden wird das Kristallisat abgesaugt und mit DMF gewaschen. Die Substanz ist chromatographisch rein.

Ausbeute: 14,6 g
Fp. 279 bis 282°
MS: 348

B. 1-(p-Acetamino-phenyl)-2,3-dimethyl-4-aminopyrazolon-5

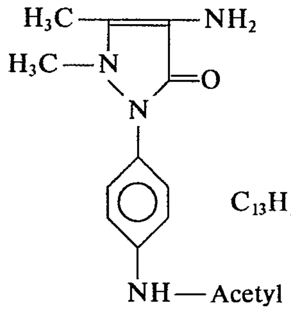

$C_{13}H_{16}N_4O_2$ MG 260

10,0 g des Produkts der Stufe A. werden in 350 ml Salzsäure (2 N) bei Raumtemperatur gelöst und anschließend auf 10°C abgekühlt. Dabei tritt die Spaltung der Benzalverbindung ein. Sie ist nach einer Stunde beendet. Zur Entfernung des Benzaldehyds wird die saure Lösung mit Chloroform extrahiert. Anschließend wird bei Raumtemperatur mit Natronlauge der pH-Wert auf 10,0 bis 11,0 eingestellt und die alkalische Lösung mit NaCl gesättigt. Anschließend erfolgt eine erneute Chloroformextraktion. Das Chloroform wird im Vakuum abgezogen.

Rückstand: 6,1 g.
Es wird aus 36 ml Äthanol umkristallisiert.

Ausbeute: 3,6 g
Fp. 210° (Z)
MS: 260
Chromatographisch einheitlich.

## Beispiel 4

### 1-(Amino-p-tolyl)-2,3-dimethyl-4-amino-pyrazolon-5

Die Synthese erfolgt gemäß nachstehendem Reaktionsschema:

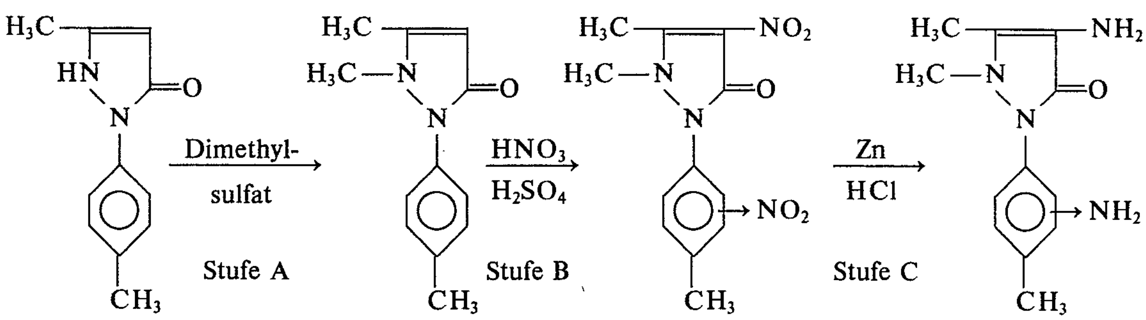


| | | | |
|---|---|---|---|
| Ausgangsmaterial (Hoechst) | | | |
| Fp 128–132° | Fp 132–134° | Fp 274–275° | Fp 165–169° |
| | MG 202 | MG 292 | MG 232 |

A. 1-(p-Tolyl)-2,3-dimethyl-pyrazolon-5

18,8 g 1-(p-Tolyl)-3-methyl-pyrazolon-5 werden bei 110 bis 115°C teilweise geschmolzen und innerhalb von 30 Minuten mit 10,5 ml Dimethylsulfat (0,11 Mol) tropfenweise versetzt. Danach wird 5 Stunden auf 170°C erhitzt. Anschließend erfolgt nach dem Abkühlen unter 100°C die Zugabe von 35 ml Wasser. Danach wird weitere 5 Stunden unter Rückfluß erhitzt. Nach dem Abkühlen auf 30°C erfolgt die Zugabe von 25 ml Natronlauge (33%). pH-Wert = 10,0 bis 11,0.

Es wird erneut 5 Stunden bei 90 bis 95°C gerührt. Danach wird auf Raumtemperatur abgekühlt und mit Chloroform (viermal je 100 ml) extrahiert. Das Chloroform wird im Vakuum abdestilliert.

Rückstand: 19,9 g
Fp. 132 – 134°
MS: 202
DC-Befund: einheitlich.

B. Dinitroverbindung des 1-(p-Tolyl)-2,3-dimethyl-pyrazolon-5

10,73 g des Produkts von Stufe A. werden bei 25°C in 60 ml Schwefelsäure (konz) eingetragen. Anschließend wird innerhalb von 2 Stunden ein Nitriergemisch von 8,05 ml Salpetersäure (65%) sowie 12 ml Schwefelsäure (konz) zugetropft unter Eiskühlung. Die Temperatur soll 5 bis 7°C betragen. Im Anschluß daran wird 30 Minuten bei Raumtemperatur nachgerührt sowie weitere 30 Minuten auf 100°C erhitzt.

Nach Abkühlen auf Raumtemperatur wird die Lösung auf 0,5 kg Eis gegeben. Nach einer weiteren Stunde wird das Kristallisat abgesaugt. Es wird aus 280 ml Eisessig umkristallisiert.

Ausbeute: 11,5 g
Fp. 274 – 275°
MS: 292

C. 1-(Amino-p-tolyl)-2,3-dimethyl-4-aminopyrazolon-5

10,3 g des Produkts von Stufe B werden in 95 ml Salzsäure (konz) bei Raumtemperatur gelöst. Unter Rühren werden zwischen 5 – 25°C (Eiswasserkühlung) innerhalb von 1,5 Stunden 30 g Zinkstaub bis zur Entfärbung der Lösung eingetragen. Danach werden bei Raumtemperatur 20 g Soda sowie insgesamt 45 g Ätznatron (Schuppen) zugesetzt. Der pH-Wert soll über 10,0 liegen.

Der dicke Brei wird bei 40°C im Vakuumschrank zur Trockne gebracht. Der trockene Rückstand wird pulverisiert und anschließend mit Chloroform 3 Stunden extrahiert.

Rückstand: 7,47 g
Die Umkristallisation erfolgt aus 27,5 ml Äthanol.

Ausbeute: 4,0 g
Fp. 165 – 169°
MS: 232
DC-Befund: einheitlich.

Beispiel 5

1-(o-Äthyl-amino-phenyl)-2,3-dimethyl-4-amino-pyrazolon-5

Die Synthese erfolgt gemäß nachstehendem Reaktionsschema:

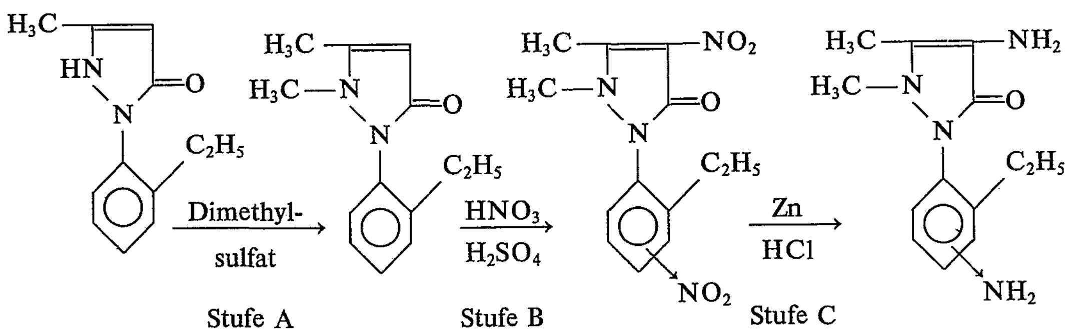


Ausgangsmaterial
(Hoechst)

| | | | |
|---|---|---|---|
| Fp 128–130° | Fp 61–61,5° | Fp 269–272° | Fp 179–181° |
| | MG 216 | MG 306 | MG 246 |

A. 1-(o-Äthyl-phenyl)-2,3-dimethyl-pyrazolon-5

Die Methylierung in 2-Position erfolgt wie in Beispiel 4, A. beschrieben.

Einsatz:

20,2 g 1-(o-Äthyl-phenyl)-3-methyl-pyrazolon-5 (0,1 Mol), 10,5 ml Dimethylsulfat (0,11 Mol). Die Substanz wird beim Erhitzen auf 170°C (5 Stunden) fest. Die zum Lösen notwendige Wassermenge beträgt 60 ml. Rückstand nach der Chloroform-Extraktion = 20,4 g. Die Reinigung erfolgt an einer Kieselgelsäule.

Ausbeute: 16,7 g
Fp. 61 – 61,5°
MS: 216
DC-Befund: einheitlich.

B. Dinitroverbindung des 1-(o-Äthyl-phenyl)-2,3-dimethyl-pyrazolon-5

Die Nitrierung wurde wie in Beispiel 4, B beschrieben durchgeführt.

Einsatz:

11,47 g 1-(o-Äthyl-phenyl)-2,3-dimethyl-pyrazolon-5
60 ml Schwefelsäure (konz)
8,05 ml Salpetersäure (65%) Nitriergemisch
12 ml Schwefelsäure (konz)
Rückstand: 14,7 g

Die Umkristallisation erfolgte aus Eisessig (390 ml).

Ausbeute: 11,07 g
Fp. 269 – 272°
MS: 306

C. 1-(Amino-o-äthyl-phenyl)-2,3-dimethyl-4-amino-pyrazolon-5

Die Reduktion der Dinitroverbindung wurde wie in Beispiel 4, C beschrieben durchgeführt.

Einsatz:

10,8 g Dinitroverbindung,
235 ml Salzsäure
36,5 g Zinkstaub
20 g Soda
100 g Ätznatron (Schuppen)

Chloroformrückstand: 7,7 g

Umfällung aus Chloroform/Diisopropyläther. Dazu werden 6,0 g des Rückstands in 140 ml Chloroform gelöst und anschließend mit 200 ml Diisopropyläther gefällt.

Ausbeute: 5,3 g
Fp. 179 – 181°
MS: 246
DC-Befund: einheitlich.

Beispiel 6

Bestimmung der $H_2O_2$-Bildung bei der Oxidation von Glycerin durch Glycerinoxidase.
Es werden zwei Reagenzien hergestellt:

Reagenz 1: 0,1 Mol/l Triäthanolamin/HCl-Puffer, pH 8,0
3,6 mMol/l bzw. 2 g/l Isotridecyläther
4,7 mMol/l bzw. 2 g/l Natriumcholat
10 mMol/l p-Chlorphenol
0,5 mMol/l aminosubstituiertes 4-Aminoantipyrin
10 U/ml Peroxidase
Reagenz 2: 500 U/ml Glycerinoxidase

Zur Durchführung der Bestimmung werden 2 ml Reagenz 1 und 0,2 ml Reagenz 2 in eine Küvette pipettiert. Die Extinktion $E_1$ wird am Photometer bei 546 nm abgelesen. Anschließend wird die Reaktion durch Zugabe von 20 µl Probe gestartet. Nach 20 Minuten Reaktionszeit wird die Extrinktion $E_2$ abgelesen. Die Inkubationstemperatur beträgt 25° C.

Die Auswertung erfolgt über eine Eichgerade, bei der die gemessene Extinktionsdifferenz $\Delta E = E_2 - E_1$ einer Glycerinstandardlösung zur Glycerinkonzentration in Beziehung gesetzt wird.

Die Konzentrationen im Testansatz betragen:

0,09 Mol/l Triäthanolamin/HCl-Puffer, pH 8,0
1,8 g/l (3,2 mMol/l) Isotridecyläther
4,3 mMol/l Natriumcholat
9 mMol/l p-Chlorphenol
0,45 mMol/l aminosubstituiertes 4-Aminoantipyrin
9 U/ml Peroxidase
45 U/ml Glycerinoxidase.

Beispiel 7

Es wurde die Farbstabilität von fünf erfindungsgemäß verwendeten Verbindungen untersucht. Als Vergleichsverbindung wurden eingesetzt:

4-Aminoantipyrin und sulfoniertes 4-Aminoantipyrin.

Mit diesen Vergleichsverbindungen und den erfindungsgemäß verwendeten Verbindungen wurden bei Einsatz von p-Chlorphenol 2,4-Dichlorphenol und Äthylhydroxytoluidin (sulfoniert) als phenolischer Kupplungspartner die Extinktion $\varepsilon$, die Stabilität des entstandenen Farbstoffs und $\lambda$max ermittelt. Das Testsystem bestand aus $H_2O_2$/POD/Farbkomponenten.

Testdurchführung:
Es wurden folgende Reagenzien verwendet:

Reagenz 1: 0,1 Mol/l Kaliumphosphatpuffer, pH 8,0
0,1 mMol/l 4-Aminoantipyrin-Derivat
1,0 mMol/l Phenolkomponente
2 U/ml POD
Reagenz 2: 0,01 Mol/l $H_2O_2$-Lösung

Bestimmungsansatz:

Meßstrahlung: Hg 546 nm;
Schichtdicke der Küvette: 1 cm;
Inkubationstemperatur: Raumtemperatur.

2,0 ml des Reagenz 1 werden in die Küvette pipettiert, 10 µl Reagenz 2 (Probe) werden zugesetzt und gemischt. Die Extinktion wird über 70 Minuten verfolgt. Pro Bestimmung wird ein Leerwert bestimmt. Hierbei wird Puffer statt der $H_2O_2$-Lösung zum Reagenz 1 zugesetzt.

Die Ergebnisse sind in der nachstehenden Tabelle zusammengefaßt. Die Prozentangabe der Farbstabilität (60 Minuten bei Raumtemperatur) bezieht sich auf die Abweichung hinsichtlich des Meßsignals, das durch $H_2O_2$ entsteht. Aus der Tabelle ist ersichtlich, daß $\lambda$max durch die erfindungsgemäß verwendeten Verbindungen nur unwesentlich beeinflußt wird, ebenso der $\varepsilon$-Wert.

Eindeutig überlegen sind die erfindungsgemäß verwendeten Verbindungen den Vergleichsverbindungen hinsichtlich der Farbstabilität.

Tabelle

| Phenolische Kupplungskomponente | Vergleich | | Erfindungsgemäß | | | | |
|---|---|---|---|---|---|---|---|
| | 4-Aminoantipyrin | 4-Aminoantipyrin (sulfoniert) | 4-Aminoantipyrinamin | 1-(Amino-o-äthylphenyl)-2,3-dimethyl-4-aminopyrazolon-(5) | 1-(p-Acetaminophenyl)-2,3-dimethyl-4-aminopyrazolon-(5) | 1-(Amino-p-tolyl)-2,3-dimethyl-4-aminopyrazolon-(5) | 1-(p-Diäthylaminophenyl)-2,3-dimethyl-4-aminopyrazolon-(5) |
| p-Chlorphenol | | | | | | | |
| ε ($cm^2/\mu Mol$) | 12 | 12 | 14 | 9 | 19 | 20 | 19 |
| Farbstabilität | +5% | +4% | +1% | ±0% | ±0% | ±0% | +1% |
| λ max | 500 | 502 | 505 | 503 | 502 | 503 | |
| 2,4-Dichlorphenol | | | | | | | |
| ε ($cm^2/\mu Mol$) | 10 | 18 | 27 | 25 | 22 | 15 | 41 |
| Farbstabilität | +2% | +7% | +1% | −1% | ±0% | +2% | +1% |
| λ max | 505 | | 507 | 508 | 507 | 508 | 502 |
| Äthylhydroxytoluidin (sulfoniert) | | | | | | | |
| ε ($cm^2/\mu Mol$) | 33 | 32 | 36 | 23 | 25 | 25 | 37 |
| Farbstabilität | +16% | −11% | −2% | −3% | −7% | −5% | −4% |
| λmax | 550 | 546 | 550 | 548 | 546 | 550 | 546 |

**Patentansprüche**

1. Verwendung einer Verbindung der allgemeinen Formel

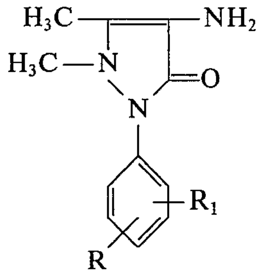

in der R die Gruppe $N(R_2)_2$, in der $R_2$ ein Wasserstoffatom, eine Alkylgruppe mit 1 bis 3 C-Atomen oder eine Acylgruppe mit 1 bis 3 C-Atomen bedeutet, darstellt und $R_1$ die gleiche Bedeutung wie R hat oder $R_1$ eine Alkylgruppe mit 1 bis 3 C-Atomen oder ein Wasserstoff ist, zusammen mit einer phenolischen Verbindung als Chromogene zur Messung der $H_2O_2$-Bildung bei enzymatischen Reaktionen in Gegenwart von Peroxidase und Puffer.

2. Verwendung einer Verbindung gemäß Anspruch 1, mit $R = NH_2$ und $R_1 = H$.

3. Verbindungen der allgemeinen Formel

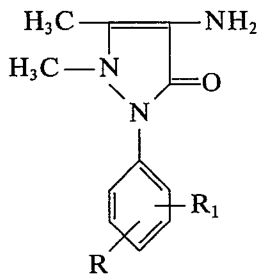

in der R die Gruppe $N(R_2)_2$ bedeutet, in der jedes $R_2$ eine Alkylgruppe mit 1 bis 3 C-Atomen oder eine Acylgruppe mit 1 bis 3 C-Atomen bedeutet und ein $R_2$ auch ein Wasserstoffatom sein kann und $R_1$ die gleiche Bedeutung wie R hat, wobei jedoch jedes $R_2$ ein H-Atom sein kann, oder $R_1$ eine Alkylgruppe mit 1 bis 3 C-Atomen oder ein Wasserstoffatom ist.

**Claims**

1. Use of a compound of the general formula:

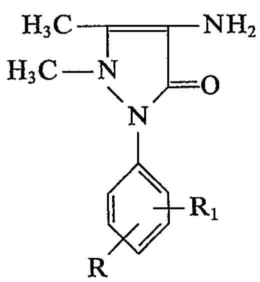

in which R represents the group $N(R_2)_2$, in which $R_2$ signifies a hydrogen atom, an alkyl group with 1 to 3 C-atoms or an acyl group with 1 to 3 C-atoms and $R_1$ has the same meaning as R or $R_1$ is an alkyl group with 1 to 3 C-atoms or a hydrogen atom, together with a phenolic compound as chromogen for the measurement of $H_2O_2$ formation in the case of enzymatic reactions in the presence of peroxidase and buffer.

2. Use of compounds according to claim 1, with $R = NH_2$ and $R_1 = H$.

3. Compounds of the general formula:

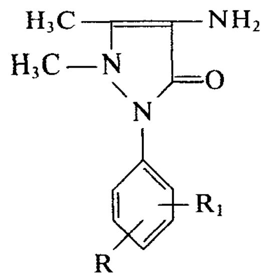

in which R signifies the group $N(R_2)_2$, in which each $R_2$ signifies an alkyl group with 1 to 3 C-atoms or an acyl group with 1 to 3 C-atoms and one $R_2$ can also be a hydrogen atom and $R_1$ has the same meaning as R, whereby, however, each $R_2$ can be an H-atom, or $R_1$ is an alkyl group with 1 to 3 C-atoms or a hydrogen atom.

## Revendications

1. Utilisation d'un composé répondant à la formule générale

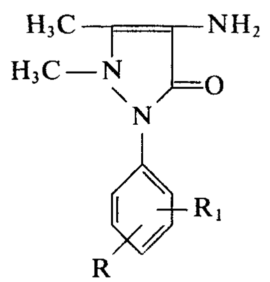

dans laquelle R désigne le groupe $N(R_2)_2$, dans lequel $R_2$ représente un atome d'hydrogène, un groupe alcoyle avec 1 à 3 atomes de C ou un groupe acyle avec 1 à 3 atomes de C et $R_1$ a la même signification que R ou $R_1$ est un groupe alcoyle avec 1 à 3 atomes de C ou un atome d'hydrogène, en même temps qu'un composé phénolique comme chromogène pour la mesure de la formation de $H_2O_2$ lors de réactions enzymatiques en présence de peroxydase et de tampon.

2. Utilisation d'un composé suivant la revendication 1, avec $R = NH_2$ et $R_1 = H$.

3. Composés répondant à la formule générale

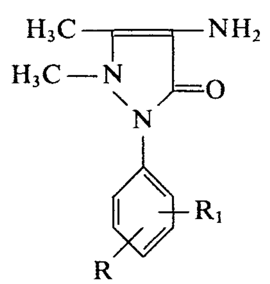

dans laquelle R désigne le groupe $N(R_2)_2$, dans lequel chaque $R_2$ représente un groupe alcoyle avec 1 à 3 atomes de C ou un groupe acyle avec 1 à 3 atomes de C et $R_2$ peut aussi être un atome d'hydrogène, et $R_1$ a la même signification que R, chacun des $R_2$ pouvant cependant être un atome H, ou $R_1$ est un groupe alcoyle avec 1 à 3 atomes de C ou un atome d'hydrogène.